(19) Europäisches Patentamt
European Patent Office
Office européen des brevets

(11) Veröffentlichungsnummer: **0 291 853 B1**

(12) **EUROPÄISCHE PATENTSCHRIFT**

(45) Veröffentlichungstag der Patentschrift: **28.07.93**

(21) Anmeldenummer: **88107593.1**

(22) Anmeldetag: **11.05.88**

(51) Int. Cl.5: **C07D 333/38**, C07D 409/06, C07D 413/14, C09B 57/00, C09B 55/00, C09B 23/00, //C09B56/00,C09B57/04, D06P1/16

# (54) Thienonverbindungen.

(30) Priorität: **19.05.87 DE 3716656**

(43) Veröffentlichungstag der Anmeldung: **23.11.88 Patentblatt 88/47**

(45) Bekanntmachung des Hinweises auf die Patenterteilung: **28.07.93 Patentblatt 93/30**

(84) Benannte Vertragsstaaten: **CH DE FR GB LI**

(56) Entgegenhaltungen:
**DD-A- 113 233**
**GB-A- 2 001 094**

**JOURNAL FÜR PRAKTISCHE CHEMIE, Band 317, Nr. 5, 1975, Seiten 861-866, Verlag Johann Ambrosius Barth, Leipzig, DD; K. GEWALD et al.: "Synthese und Reaktionen von 2-Hydroxy-3-cyan-thiophenen"**

**SYNTHESIS, Band 2, Februar 1985, Seiten 172-174, Georg Thieme Verlag, Stuttgart, DE; E. CHAUMANN et al.: "Synthesis of alkyl 2-Alkenimidothiates from alkyl 2-(Diethoxyphosphinyl)-alkanimidothioates and aldehydes via Wittig-Horner olefination"**

(73) Patentinhaber: **BASF Aktiengesellschaft**
**Carl-Bosch-Strasse 38**
**W-6700 Ludwigshafen(DE)**

(72) Erfinder: **Hansen, Guenter, Dr.**
**Alwin-Mittasch-Platz 8**
**W-6700 Ludwigshafen(DE)**
Erfinder: **Dix, Johannes Peter, Dr.**
**Ludwigshafener Strasse 125**
**W-6708 Neuhofen(DE)**
Erfinder: **Reichelt, Helmut, Dr.**
**Weinbachstrasse 20**
**W-6701 Niederkirchen(DE)**
Erfinder: **Hayashi, Masahiro**
**Waltraudenstrasse 35**
**W-6700 Ludwigshafen(DE)**

EP 0 291 853 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Thien-2-onverbindungen, die in Ringposition 5 eine Alkyliden- oder eine Iminogruppe aufweisen, sowie ihre Verwendung zum Färben von Fasern.

Aus der DE-A-2 732 221 sind Farbstoffe auf Basis von 1,3-Thiazolderivaten, die in Ringposition 2 eine Alkylidengruppe und in Ringposition 5 eine Alkyliden- oder eine Iminogruppe aufweisen, bekannt.

Es wurden nun Thienonverbindungen der Formel I

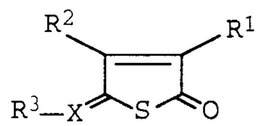 (I),

gefunden, in der

| | |
|---|---|
| $R^1$ | Cyano, $C_1$-$C_4$-Alkoxycarbonyl, Carbamoyl oder $C_1$-$C_4$-Mono- oder Dialkylcarbamoyl, |
| $R^2$ | Wasserstoff, $C_1$-$C_8$-Alkyl, gegebenenfalls durch $C_1$-$C_4$-Alkyl, $C_1$-$C_4$-Alkoxy, Chlor, Brom oder Nitro substituiertes Phenyl, Furyl, Thienyl oder Halogen, |
| X | Stickstoff oder den Rest $CR^4$, in dem $R^4$ für Wasserstoff, $C_1$-$C_4$-Alkyl, $C_1$-$C_4$-Alkoxycarbonyl, Cyano oder gegebenenfalls durch $C_1$-$C_4$-Alkyl, $C_1$-$C_4$-Alkoxy, Chlor, Brom oder Nitro substituiertes Phenyl steht, und |
| $R^3$ | einen Rest der Formel |

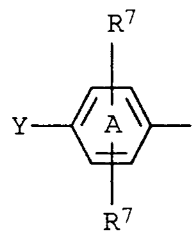 , 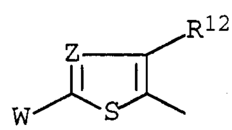 , 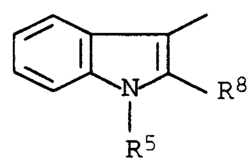 ,

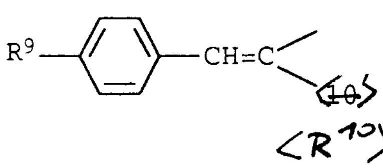 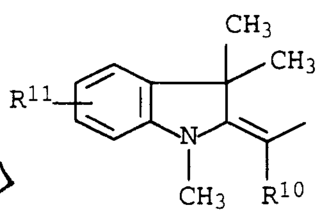 oder 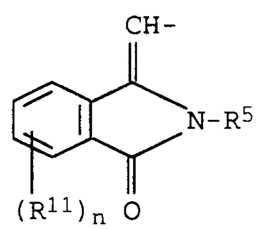

bedeuten, wobei

der Ring A gegebenenfalls benzoanelliert ist,

| | |
|---|---|
| Y | für $NR^5R^6$ oder $OR^8$, |
| $R^5$ und $R^6$ | gleich oder verschieden sind und unabhängig voneinander jeweils für Wasserstoff, $C_1$-$C_8$-Alkyl, das gegebenenfalls durch Hydroxy, Chlor, Cyano, $C_1$-$C_4$-Alkoxy, $C_1$-$C_4$-Alkanoyloxy, $C_1$-$C_4$-Alkoxycarbonyloxy oder Phenyl substituiert ist, Allyl, $C_5$-$C_7$-Cycloalkyl oder Phenyl oder zusammen mit dem sie verbindenden Stickstoffatom für einen 5- oder 6-gliedrigen gesättigten heterocyclischen Ring, |
| $R^8$ | für $C_1$-$C_4$-Alkyl oder Phenyl, |
| $R^7$ | unabhängig voneinander jeweils für Wasserstoff, Chlor, Brom, Hydroxy, $C_1$-$C_4$-Alkyl, $C_1$-$C_4$-Alkoxy, gegebenenfalls durch Chlor, $C_1$-$C_4$-Alkoxy oder Phenoxy substituiertes $C_1$-$C_4$-Alkanoylamino, Benzoylamino, $C_1$-$C_4$-Mono- oder Dialkylaminosulfonylamino, $C_1$-$C_4$-Alkanoyloxy oder Phenylsulfonyloxy, |
| $R^9$ | für Wasserstoff, $C_1$-$C_4$-Dialkylamino oder $C_1$-$C_4$-Alkoxy, |
| $R^{10}$ | für Wasserstoff oder Cyano, |
| $R^{11}$ | für Wasserstoff, Chlor, Brom oder $C_1$-$C_4$-Alkyl, |

$R^{12}$ für Wasserstoff, Chlor, Brom, $C_1$-$C_8$-Alkyl, gegebenenfalls durch $C_1$-$C_4$-Alkyl, $C_1$-$C_4$-Alkoxy, Chlor, Brom oder Nitro substituiertes Phenyl oder Thienyl,

W für den Rest $NR^5R^6$ oder

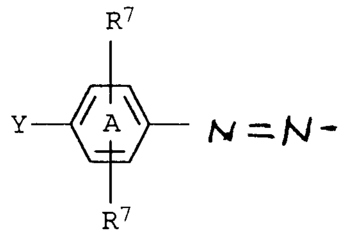

wobei $R^5$, $R^6$, $R^7$, Y und der Ring A jeweils die obengenannte Bedeutung besitzen,

Z für Stickstoff oder den Rest $CR^1$, worin $R^1$ die obengenannte Bedeutung besitzt, und

n für 1, 2, 3 oder 4 stehen,

mit der Maßgabe, daß wenn $R^1$ für Cyano, X für den Rest CH und $R^3$ für 4-Dimethylaminophenyl oder Styryl stehen, $R^2$ nicht Methyl oder Phenyl bedeutet, und daß wenn $R^1$ für Cyano, X für den Rest CH und $R^3$ für 4-Methoxyphenyl stehen, $R^2$ nicht Phenyl bedeutet.

3-Cyanothien-2-one, die in Ringposition 4 Phenyl und in Ringposition 5 Benzyliden, 4-Methoxybenzyliden, 4-Dimethylaminobenzyliden oder 3-Phenyl-prop-2-enyliden sowie in Ringposition 4 Methyl und in Ringposition 5 4-Dimethylaminobenzyliden oder 3-Phenyl-prop-2-enyliden als Substituenten aufweisen, sind bekannt und in J. Prakt. Chem. 317, 861 (1975), sowie in der Dissertation von M. Hentschel, Technische Universität Dresden, 28.01.1975, beschrieben.

Alle in den obengenannten Resten der Formel I auftretenden Alkylgruppen können sowohl gradkettig als auch verzweigt sein.

In Formel I sind $R^1$ und $R^4$ beispielsweise Methoxycarbonyl, Ethoxycarbonyl, Propoxycarbonyl, Isopropoxycarbonyl, Butoxycarbonyl oder sec-Butoxcarbonyl.

$R^1$ ist weiterhin beispielsweise Methylcarbamoyl, Ethylcarbamoyl, Propylcarbamoyl, Isopropylcarbamoyl, Butylcarbamoyl, Dimethylcarbamoyl, Diethylcarbamoyl, Dipropylcarbamoyl, Diisopropylcarbamoyl, Dibutylcarbamoyl oder N-Methyl-N-ethylcarbamoyl.

$R^2$, $R^5$, $R^6$, $R^7$, $R^8$, $R^{11}$ und $R^{12}$ sind beispielsweise Methyl, Ethyl, Propyl, Isopropyl, Butyl, Isobutyl, sec-Butyl oder tert-Butyl.

$R^2$, $R^5$, $R^6$ und $R^{12}$ sind weiterhin beispielsweise Pentyl, Isopentyl, Neopentyl, tert-Pentyl, Hexyl, 2-Methylpentyl, Heptyl, 2-Methylhexyl, Octyl oder 2-Ethylhexyl.

$R^2$ bedeutet weiterhin beispielsweise Fluor, Chlor oder Brom.

$R^5$ und $R^6$ sind weiterhin beispielsweise 2-Hydroxyethyl, 2-Chlorethyl, 2-Cyanoethyl, 2-Methoxyethyl, 2-Ethoxethyl, 2-Propoxyethyl, 2-Butoxyethyl, 2-Formyloxyethyl, 2-Acetyloxyethyl, 2-Propionyloxyethyl, 2-Butyryloxyethyl, 2-Methoxycarbonyloxyethyl, 2-Ethoxycarbonyloxyethyl, 2-Propoxycarbonyloxyethyl, 2-Butoxycarbonyloxyethyl, 2-Methoxypropyl, 3-Methoxypropyl, Benzyl, 1- oder 2-Phenylethyl, Phenyl, Cyclopentyl, Cyclohexyl oder Cycloheptyl.

$R^5$ und $R^6$ stehen weiterhin mit den sie verbindenden Stickstoffatom z. B. für Pyrrolidino, Piperidino, Morpholino, Piperazino oder N-($C_1$-$C_4$-Alkyl )piperazino, wie N-Methyl- oder N-Ethylpiperazino.

Weiterhin bedeuten $R^7$ und $R^9$ beispielsweise Methoxy, Ethoxy, Propoxy, Isopropoxy, Butoxy, Isobutoxy oder sec-Butoxy.

$R^7$ bedeutet weiterhin beispielsweise Formylamino, Acetylamino, Propionylamino, Butyrylamino, Isobutyrylamino, Chloracetylamino, Methoxyacetylamino, Ethoxyacetylamino, Phenoxyacetylamino, 3-Chlorpropionylamino, 3-oder 4-Methoxybutyrylamino, Methyl- oder Ethylaminosulfonylamino, Dimethylaminosulfonylamino, Diethylaminosulfonylamino, Formyloxy, Acetyloxy, Propionyloxy, Butyryloxy oder Isobutyryloxy.

$R^9$ bedeutet weiterhin beispielsweise Methylamino, Ethylamino, Propylamino, Isopropylamino, Butylamino, Dimethylamino, Diethylamino, Dipropylamino, Diisopropylamino oder N-Methyl-N-ethylamino.

$R^2$, $R^4$ und $R^{12}$ sind weiterhin beispielsweise 2- oder 4-Methylphenyl, 4-Ethylphenyl, 4-Isopropylphenyl, 2,4-Dimethylphenyl, 2- oder 4-Methoxyphenyl, 4-Ethoxyphenyl, 2,4-Dimethoxyphenyl, 2- oder 4-Chlorphenyl, 2-oder 4-Bromphenyl, 2,6-Dichlorphenyl oder 3-Nitrophenyl.

$R^3$ entspricht unter anderem beispielsweise der Formel

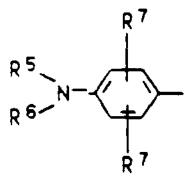
,

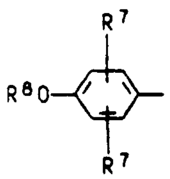
,

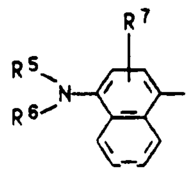
,

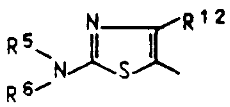
,

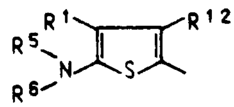
,

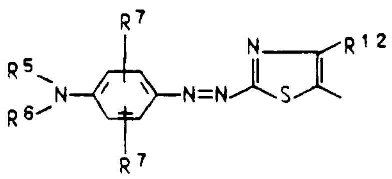
,

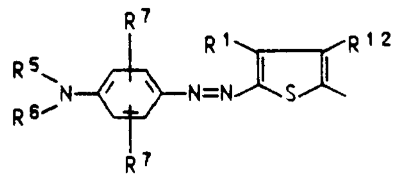

oder

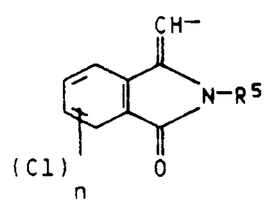
,

wobei $R^1$, $R^5$, $R^6$, $R^7$, $R^8$, $R^{12}$ und n jeweils die obengenannte Bedeutung besitzen.

Bevorzugt sind solche Thienonverbindungen der Formel I, in der

$R^1$ Cyano oder Carbamoyl,

$R^2$ $C_1$-$C_4$-Alkyl, gegebenenfalls durch Methyl, Ethyl, Methoxy, Ethoxy, Chlor oder Brom substituiertes Phenyl oder Thienyl, X Stickstoff oder den Rest $CR^4$, in dem $R^4$ für Wasserstoff oder Cyano steht, und

$R^3$ einen Rest der Formel

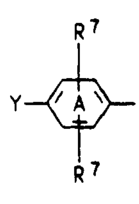

bedeuten, wobei Y, $R^7$ und der Ring A jeweils die obengenannte Bedeutung besitzen.

Insbesondere bevorzugt sind Thienonverbindungen der Formel I, in der $R^1$ Cyano, $R^2$ Methyl, gegebenenfalls durch Methoxy substituiertes Phenyl oder Thienyl, X Stickstoff oder den Rest $CR^4$, in dem $R^4$ für Wasserstoff oder Cyano steht, und

$R^3$ einen Rest der Formel

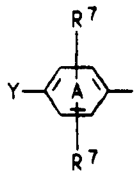

bedeuten, wobei der Ring A nicht benzoanelliert ist und Y und $R^7$ jeweils die obengenannte Bedeutung besitzen.

Zur Herstellung der Verbindungen der Formel I kann man beispielsweise ein 2-Hydroxythiophen der Formel II

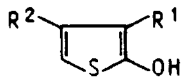

(II),

in der $R^1$ und $R^2$ jeweils die obengenannte Bedeutung besitzen, mit einer Verbindung der Formel III

$R^3 - X = 0$ (III),

in der $R^3$ und X [mit Ausnahme von X = C(CN)] die obengenannte Bedeutung besitzen, kondensieren. Wenn dabei die Verbindungen der Formel III Aldehyde oder Ketone darstellen, kann es in manchen Fällen vorteilhaft sein, diese in Form ihrer Acetale einzusetzen.

Die Kondensation wird zweckmäßig in einem inerten Lösungsmittel bei einer Temperatur von 20 bis 160 °C, vorzugsweise 20 bis 100 °C, durchgeführt. Als Lösungsmittel eignen sich z.B. Methanol, Ethanol, Propanol, Isopropanol, Butanol, Isobutanol, Methylglykol, Dioxan, Aceton, Acetonitril, N,N-Dimethylformamid, N-Methylpyrrolidon, Toluol, Xylol, Chlorbenzol oder Nitrobenzol. Vorzugsweise wird in Alkoholen, z.B. in Propanol gearbeitet.

Die Kondensation kann auch unter Hinzufügen von sauren oder basischen Katalysatoren erfolgen. Das während der Reaktion entstehende Wasser kann bei Verwendung geeigneter Lösungsmittel, z.B. Toluol, aus dem Reaktionsgemisch azeotrop entfernt werden. Es ist aber auch möglich, die Reaktion in Gegenwart von Wasser durchzuführen.

Als saure Katalysatoren eignen sich z. B. Mineralsäuren, wie Chlorwasserstoffsäure oder Schwefelsäure, Carbonsäuren, wie Ameisensäure, Essigsäure oder Trichloressigsäure, Phosphoroxychlorid, Phosphorpentachlorid, Phosphortrichlorid oder Thionylchlorid.

Als basische Katalysatoren sind z. B. Ammoniumacetat oder Amine, wie Piperidin, Pyrrolidin, Pyridin, Triethylamin oder Triethanolamin, zu nennen.

Zur Herstellung derjenigen Verbindungen der Formel I, in der X für den Rest C(CN) steht, kann man solche Verbindungen der Formel I, in der X für den Rest CH steht, mit Metallcyaniden, wie Natriumcyanid oder Kaliumcyanid, umsetzen und in einer Folgereaktion mit geeigneten Oxidationsmitteln, z.B. mit Brom, $K_3[Fe(CN)_6]$, $Pb(OAc)_4$ oder $FeCl_3 x 6H_2O$ reoxidieren. Diese Cyanierungs-Methode ist in Dyes and Pigments 1, 3 (1980), bereits für Cumarinderivate beschrieben.

Einige Verbindungen der Formel II sind aus den obengenannten Referaten bekannt. Weiterhin beschreibt die DD-A-113 233 ein Verfahren zur Herstellung von 2-Hydroxy-3-cyanothiophenen, deren Ringposition 5 unsubstituiert ist (z. B. 2-Hydroxy-3-cyano-4-phenylthiophen).

Weitere Einzelheiten der Herstellung können den Beispielen entnommen werden.

Es wurde weiterhin gefunden, daß die Thienonverbindungen der Formel Ia

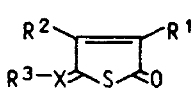

(Ia),

in der

| | |
|---|---|
| $R^1$ | Cyano, $C_1$-$C_4$-Alkoxycarbonyl, Carbamoyl oder $C_1$-$C_4$-Mono- oder Dialkylcarbamoyl, |
| $R^2$ | Wasserstoff, $C_1$-$C_8$-Alkyl, gegebenenfalls durch $C_1$-$C_4$-Alkyl, $C_1$-$C_4$-Alkoxy, Chlor, Brom |

oder Nitro substituiertes Phenyl, Furyl, Thienyl oder Halogen,

X Stickstoff oder den Rest $CR^4$, in dem $R^4$ für Wasserstoff, $C_1$-$C_4$-Alkyl, $C_1$-$C_4$-Alkoxycarbonyl, Cyano oder gegebenenfalls durch $C_1$-$C_4$-Alkyl, $C_1$-$C_4$-Alkoxy, Chlor, Brom oder Nitro substituiertes Phenyl steht, und

$R^3$ einen Rest der Formel

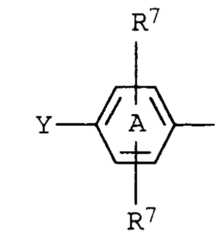 , 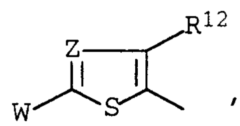 , 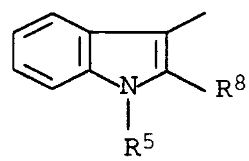 ,

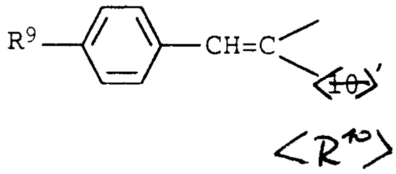 , 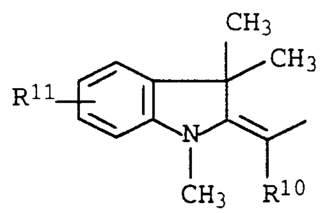 oder 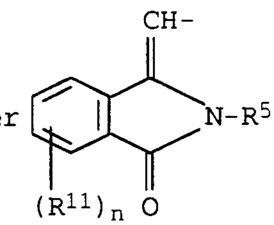

bedeuten, wobei

der Ring A gegebenenfalls benzoanelliert ist,

Y für $NR^5R^6$ oder $OR^8$,

$R^5$ und $R^6$ gleich oder verschieden sind und unabhängig voneinander jeweils für Wasserstoff, $C_1$-$C_8$-Alkyl, das gegebenenfalls durch Hydroxy, Chlor, Cyano, $C_1$-$C_4$-Alkoxy, $C_1$-$C_4$-Alkanoyloxy, $C_1$-$C_4$-Alkoxycarbonyloxy oder Phenyl substituiert ist, Allyl, $C_5$-$C_7$-Cycloalkyl oder Phenyl oder zusammen mit dem sie verbindenen Stickstoffatom für einen 5- oder 6-gliedrigen gesättigten heterocyclischen Ring,

$R^8$ für $C_1$-$C_4$-Alkyl oder Phenyl,

$R^7$ unabhängig voneinander jeweils für Wasserstoff, Chlor, Brom, Hydroxy, $C_1$-$C_4$-Alkyl, $C_1$-$C_4$-Alkoxy, gegebenenfalls durch Chlor, $C_1$-$C_4$-Alkoxy oder Phenoxy substituiertes $C_1$-$C_4$-Alkanoylamino, Benzoylamino, $C_1$-$C_4$-Mono- oder Dialkylaminosulfonylamino, $C_1$-$C_4$-Alkanoyloxy oder Phenylsulfonyloxy,

$R^9$ für Wasserstoff, $C_1$-$C_4$-Dialkylamino oder $C_1$-$C_4$-Alkoxy,

$R^{10}$ für Wasserstoff oder Cyano,

$R^{11}$ für Wasserstoff, Chlor, Brom oder $C_1$-$C_4$-Alkyl,

$R^{12}$ für Wasserstoff, Chlor, Brom, $C_1$-$C_8$-Alkyl, gegebenenfalls durch $C_1$-$C_4$-Alkyl, $C_1$-$C_4$-Alkoxy, Chlor, Brom oder Nitro substituiertes Phenyl oder Thienyl,

W für den Rest $NR^5R^6$ oder

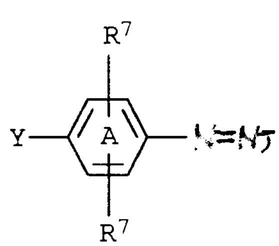

wobei $R^5$, $R^6$, $R^7$, Y und der Ring A jeweils die obengenannte Bedeutung besitzen,

Z für Stickstoff oder den Rest $CR^1$, worin $R^1$ die obengenannte Bedeutung besitzt, und

n für 1, 2, 3 oder 4 stehen,

als Farbstoffe geeignet sind. Sie eignen sich insbesondere zum Färben von Fasern, wie Polyester, Polyamide, Celluloseester oder Mischgewebe aus Polyester und Cellulosefasern.

Man erhält brillante Färbungen in gelben bis grünstichig blauen Tönen mit guten Echtheiten, insbesondere auf Polyestern. Darüberhinaus zeichnen sich die Farbstoffe durch ihre große Farbstärke aus.

Die nachfolgenden Beispiele sollen die Erfindung näher erläutern.

A) Herstellung der in Ringposition 5 unsubstituierten 2-Hydroxythiophene

Beispiel 1

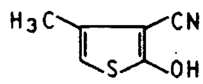

Zu 18,5 g 2-Amino-3-ethoxycarbonyl-4-methylthiophen in 200 ml Toluol wurde bei einer Temperatur von 80 bis 85 °C unter Stickstoffatmosphäre eine Lösung von 3,9 g Natrium in 100 ml Ethanol innerhalb 1 Stunde getropft. Danach wurden 200 ml eines Ethanol/Toluol-Gemisches aus dem Reaktionsgemisch bei 85 bis 100 °C abdestilliert. Der verbleibende Niederschlag wurde bei Raumtemperatur abgesaugt, mit wenig Toluol gewaschen und in 250 ml Wasser gelöst. Mit konzentrierter Salzsäure wurde ein pH-Wert von 1 eingestellt, nach Zugabe von 100 ml Methylenchlorid die organische Phase abgetrennt und eingeengt.

Ausbeute: 11,0 g anfangs öliges, später kristallines 2-Hydroxy-3-cyano-4-methylthiophen (79 % d. Th.) Schmp.: 52-53 °C (Petrolether).

Die in Tabelle 1 aufgeführten 3-Cyano-2-hydroxythiophene der Formel

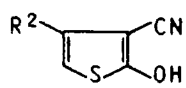

werden in analoger Weise erhalten.

Tabelle 1

| Beispiel | $R^2$ |
|---|---|
| 2 | 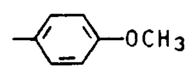 |
| 3 | 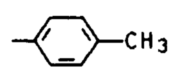 |
| 4 | 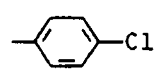 |
| 5 | 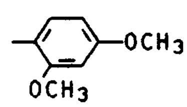 |
| 6 | 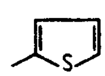 |

Beispiel 7

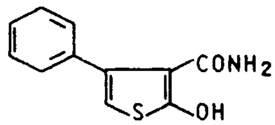

Zu 50 g Polyphosphorsäure wurden bei 90 °C unter Rühren 2,01 g 3-Cyano-2-hydroxy-4-phenylthiophen gegeben. Die Reaktionsmischung wurde 4 Stunden bei dieser Temperatur gerührt. Nach dem Erkalten wurden 50 g Eis zugesetzt, eine Stunde gerührt, der Niederschlag abgesaugt und gründlich mit Wasser gewaschen. Man erhielt 1,6 g (73 % d.Th.) 3-Carbamoyl-2-hydroxy-4-phenylthiophen mit Schmp. 108 - 110 °C.

Die in Tabelle 2 aufgeführten 3-Carbamoyl-2-hydroxythiophene der Formel

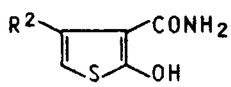

werden in analoger Weise erhalten.

Tabelle 2

| Beispiel | $R^2$ |
|---|---|
| 8 | 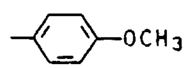 |
| 9 | 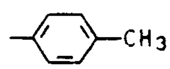 |
| 10 | $-CH_3$ |

B) Herstellung der erfindungsgemäßen Thienone

Beispiel 11

2,01 g 3-Cyano-2-hydroxy-4-phenylthiophen und 3,20 g 3-Methoxy-4-nitroso-N,N-dihexylanilin wurden in 60 ml n-Propanol 12 Stunden bei 25 °C gerührt. Man gab auf 500 ml Wasser, rührte 15 Minuten nach, saugte ab und trocknete. Man erhielt 4,9 g Farbstoff (97 % d. Th.), der Polyesterfasern in einem brillanten neutralblauen Ton färbt.
$\lambda_{max}$ ($CH_2Cl_2$): 602 nm

Beispiel 12

2,01 g 3-Cyano-2-hydroxy-4-phenylthiophen und 2,31 g 4-Nitroso-N-butyl-N-(2-cyanoethyl)anilin wurden in 50 ml n-Propanol 10 Stunden bei 25 °C gerührt. Nach Zugabe von 100 ml Wasser wurde der Farbstoff abgesaugt, mit Wasser gewaschen und getrocknet.

Ausbeute: 4,00 g (97 % d. Th.), Schmp. 164 - 166 °C (Propanol), $\lambda_{max}$ ($CH_2Cl_2$): 556 nm.
Der Farbstoff färbt Polyester in violetten Tönen.

Beispiel 13

1,74 g 3-Cyano-2-hydroxy-4-methylthiophen und 1,39 g 4-N,N-Diethylaminobenzaldehyd wurden 14 Stunden in 40 ml n-Propanol bei 20 °C gerührt. Der Niederschlag wurde abgesaugt und mit wenig Propanol gewaschen. Man erhielt 2,85 g Farbstoff (96 % d. Th.), der Polyesterfasermaterial in brillanten, scharlachroten Tönen färbt.
Schmp.: 205 - 207 °C (Propanol), $\lambda_{max}$ ($CH_2Cl_2$): 521 nm

Die in Tabelle 3 aufgeführten Farbstoffe werden in analoger Weise erhalten. Es sind dabei jeweils die beiden Ausgangskomponenten angegeben.

Tabelle 3

| Beispiel | $R^3$—X=O | 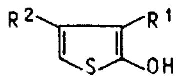 | Farbton auf Polyester | $\lambda_{max}$ [nm] ($CH_2Cl_2$) |
|---|---|---|---|---|
| 14 | 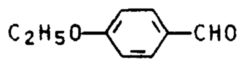 | 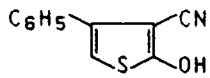 | gelb | 430 |
| 15 | 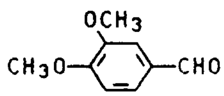 | 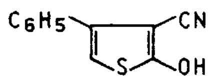 | gelb | 445 |
| 16 | 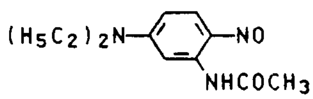 | 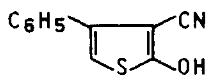 | blau | 612 |
| 17 | 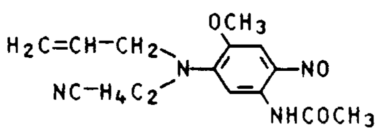 | 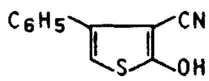 | blau | |
| 18 | 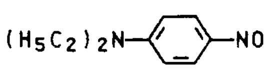 | 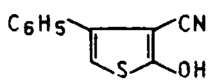 | blau | 589 |
| 19 | 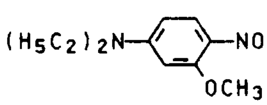 | 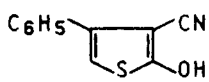 | blau | 597 |
| 20 | 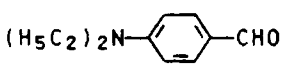 | 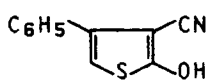 | rot | |
| 21 | 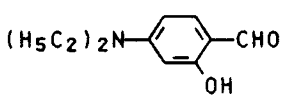 | 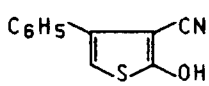 | rot | |
| 22 | 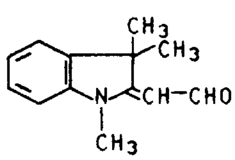 | 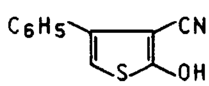 | rotviolett | 552 |
| 23 | 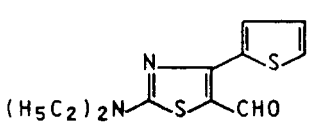 | 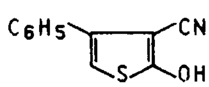 | violett | 572 |

| Beispiel | $R^3-X=O$ | 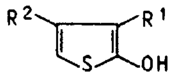 | Farbton auf Polyester | $\lambda_{max}$ [nm] ($CH_2Cl_2$) |
|---|---|---|---|---|
| 24 | 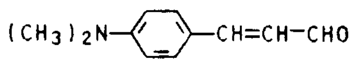 | 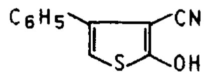 | blau | 578 |
| 25 | 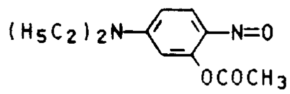 | 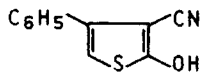 | rotstichig blau | |
| 26 | 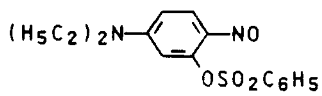 | 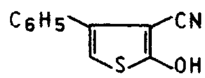 | rotstichig blau | |
| 27 | 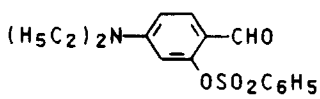 | 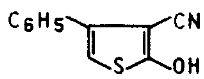 | rot | |
| 28 | 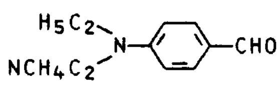 | 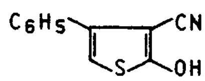 | scharlach | |
| 29 | 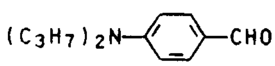 | 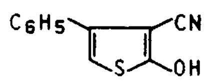 | rot | |
| 30 | 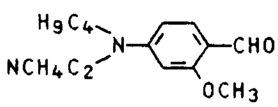 | 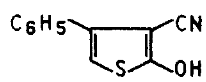 | rot | |
| 31 | 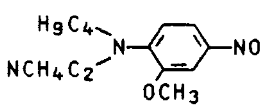 | 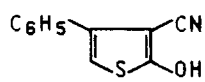 | rotstichig blau | |
| 32 | 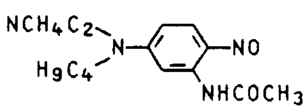 | 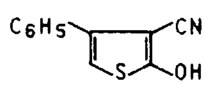 | blau | |
| 33 | 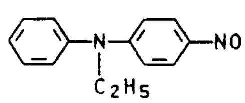 | 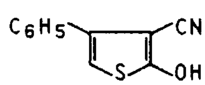 | blau | |
| 34 | 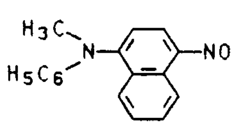 | 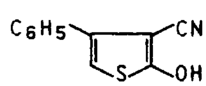 | blau | 576 |

| Beispiel | $R^3-X=O$ | 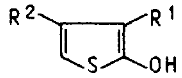 | Farbton auf Polyester | $\lambda_{max}$ [nm] ($CH_2Cl_2$) |
|---|---|---|---|---|
| 35 | 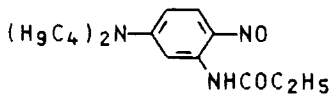 | 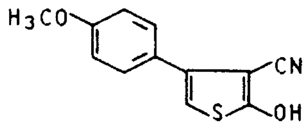 | grünstichig blau | |
| 36 | 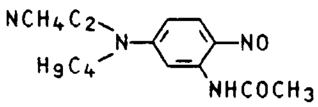 | 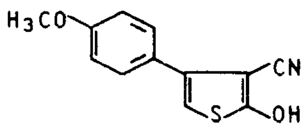 | blau | |
| 37 | 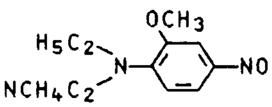 | 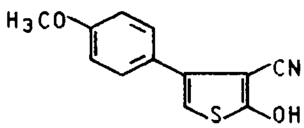 | blau | |
| 38 | 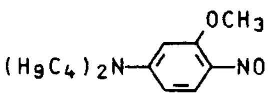 | 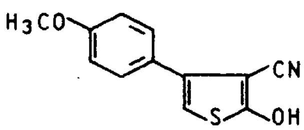 | blau | |
| 39 | 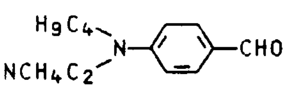 | 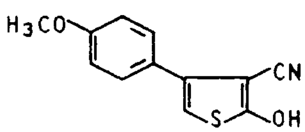 | rot | |
| 40 | 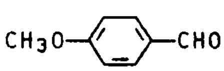 | 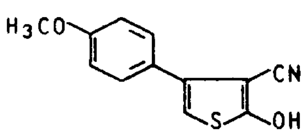 | gelb | |
| 41 | 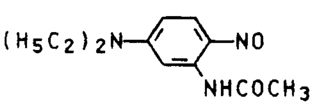 | 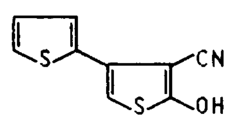 | grünstichig blau | |
| 42 | 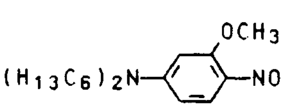 | 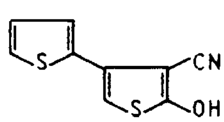 | grünstichig blau | |

| Beispiel | $R^3-X=O$ | 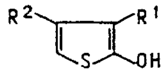 | Farbton auf Polyester | $\lambda_{max}$ [nm] ($CH_2Cl_2$) |
|---|---|---|---|---|
| 43 | 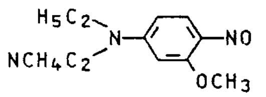 | 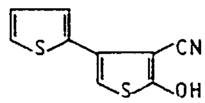 | blau | |
| 44 | 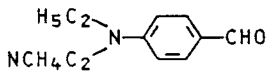 | 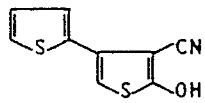 | rot | |
| 45 | 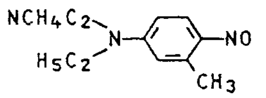 | 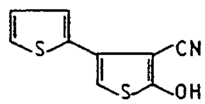 | blau | |
| 46 | 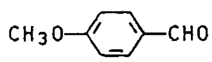 | 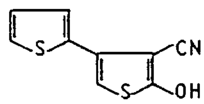 | gelb | |
| 47 | 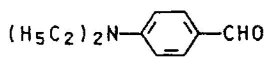 | 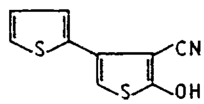 | violett | |
| 48 | 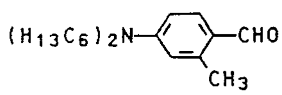 | 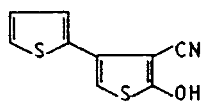 | rot | |
| 49 | 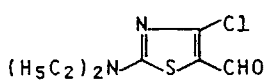 | 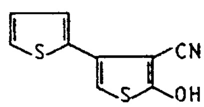 | rot | |
| 50 | 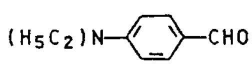 | 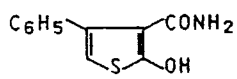 | rot | |
| 51 | 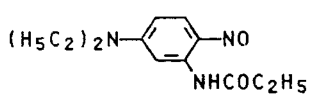 | 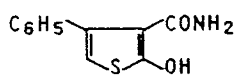 | blau | |
| 52 | 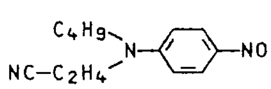 | 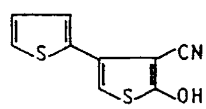 | blau | 577 |

Beispiel 53

1,66 g des Farbstoffs aus Beispiel 29 wurden in 50 ml N,N-Dimethylformamid mit 0,5 g KCN versetzt und 1 Stunde bei Raumtemperatur gerührt. Nach Zugabe von 1,35 g $FeCl_3 x 6H_2O$ wurde weitere 8 Stunden nachgerührt. Die Reaktionsmischung wurde auf 150 ml Eiswasser gegossen, der Niederschlag abfiltriert und

mit Wasser gewaschen. Man erhielt 1,7 g (95 % d. Th.) des Farbstoffs der Formel

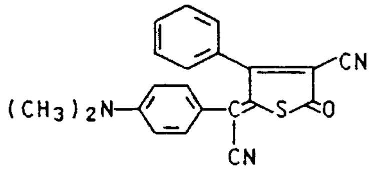

der Polyester in blauen Tönen färbt. ($\lambda_{max}$ ($CH_2Cl_2$): 599nm).

Die in Tabelle 4 aufgeführten Farbstoffe der Formel

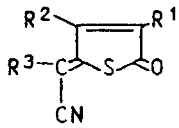

werden in analoger Weise erhalten

Tabelle 4

| Beispiel | $R^3$ | $R^2$ | $R^1$ | Farbton auf Polyester |
|---|---|---|---|---|
| 54 | 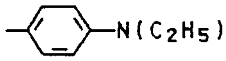 | $C_6H_5$ | CN | blau |
| 55 | 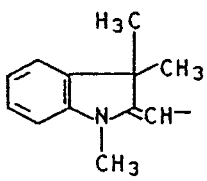 | $C_6H_5$ | CN | blau |
| 56 | 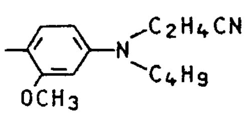 | $C_6H_5$ | CN | blau |
| 57 | 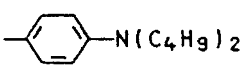 | 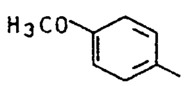 | CN | blau |

| Beispiel | $R^3$ | $R^2$ | $R^1$ | Farbton auf Polyester |
|---|---|---|---|---|
| 58 | 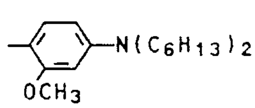 | 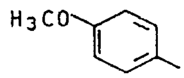 | CN | blau |
| 59 | 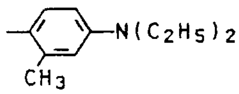 | 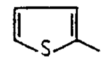 | CN | blau |
| 60 | 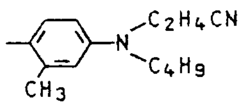 | 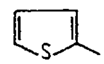 | CN | blau |

## Patentansprüche

1. Thienonverbindungen der Formel I

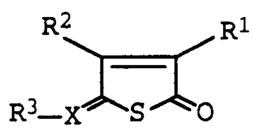 (I),

in der

$R^1$ Cyano, $C_1$-$C_4$-Alkoxycarbonyl, Carbamoyl oder $C_1$-$C_4$-Monooder Dialkylcarbamoyl,

$R^2$ Wasserstoff, $C_1$-$C_8$-Alkyl, gegebenenfalls durch $C_1$-$C_4$-Alkyl, $C_1$-$C_4$-Alkoxy, Chlor, Brom oder Nitro substituiertes Phenyl, Furyl, Thienyl oder Halogen,

X Stickstoff oder den Rest $CR^4$, in dem $R^4$ für Wasserstoff, $C_1$-$C_4$-Alkyl, $C_1$-$C_4$-Alkoxycarbonyl, Cyano oder gegebenenfalls durch $C_1$-$C_4$-Alkyl, $C_1$-$C_4$-Alkoxy, Chlor, Brom oder Nitro substituiertes Phenyl steht, und

$R^3$ einen Rest der Formel

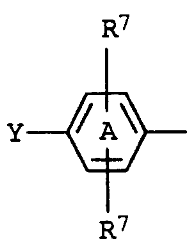 , 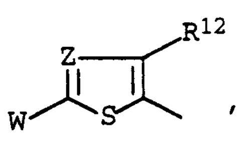 , 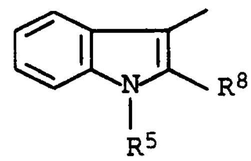 ,

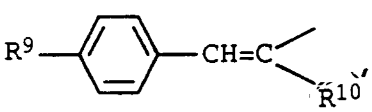 , 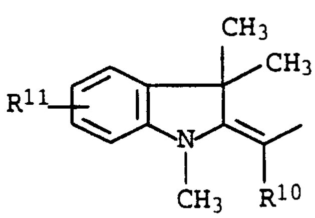 oder 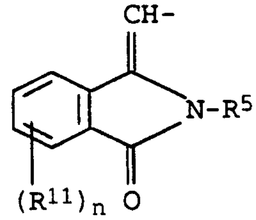

bedeuten, wobei
der Ring A gegebenenfalls benzoanelliert ist,

Y für $NR^5R^6$ oder $OR^8$,

$R^5$ und $R^6$ gleich oder verschieden sind und unabhängig voneinander jeweils für Wasserstoff, $C_1$-$C_8$-Alkyl, das gegebenenfalls durch Hydroxy, Chlor, Cyano, $C_1$-$C_4$-Alkoxy, $C_1$-$C_4$-Alkanoyloxy, $C_1$-$C_4$-Alkoxycarbonyloxy oder Phenyl substituiert ist, Allyl, $C_5$-$C_7$-Cycloalkyl oder Phenyl oder zusammen mit dem sie verbindendenen Stickstoffatom für einen 5-oder 6-gliedrigen gesättigten heterocyclischen Ring,

$R^8$ für $C_1$-$C_4$-Alkyl oder Phenyl,

$R^7$ unabhängig voneinander jeweils für Wasserstoff, Chlor, Brom, Hydroxy, $C_1$-$C_4$-Alkyl, $C_1$-$C_4$-Alkoxy, gegebenenfalls durch Chlor, $C_1$-$C_4$-Alkoxy oder Phenoxy substituiertes $C_1$-$C_4$-Alkanoylamino, Benzoylamino, $C_1$-$C_4$-Mono- oder Dialkylaminosulfonylamino, $C_1$-$C_4$-Alkanoyloxy oder Phenylsulfonyloxy,

$R^9$ für Wasserstoff, $C_1$-$C_4$-Dialkylamino oder $C_1$-$C_4$-Alkoxy,

$R^{10}$ für Wasserstoff oder Cyano,

$R^{11}$ für Wasserstoff, Chlor, Brom oder $C_1$-$C_4$-Alkyl,

$R^{12}$ für Wasserstoff, Chlor, Brom, $C_1$-$C_8$-Alkyl, gegebenenfalls durch $C_1$-$C_4$-Alkyl, $C_1$-$C_4$-Alkoxy, Chlor, Brom oder Nitro substituiertes Phenyl oder Thienyl,

W für den Rest $NR^5R^6$ oder

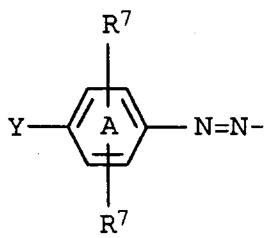,

wobei $R^5$, $R^6$, $R^7$, Y und der Ring A jeweils die obengenannte Bedeutung besitzen,

Z für Stickstoff oder den Rest $CR^1$, worin $R^1$ die obengenannte Bedeutung besitzt, und

n für 1, 2, 3 oder 4 stehen,

mit der Maßgabe, daß wenn $R^1$ für Cyano, X für den Rest CH und $R^3$ für 4-Dimethylaminophenyl oder Styryl stehen, $R^2$ nicht Methyl oder Phenyl bedeutet, und daß wenn $R^1$ für Cyano, X für den Rest CH und $R^3$ für 4-Methoxyphenyl stehen, $R^2$ nicht Phenyl bedeutet.

**2.** Thienonverbindungen gemäß Anspruch 1, dadurch gekennzeichnet, daß $R^1$ Cyano oder Carbamoyl, $R^2$ $C_1$-$C_4$-Alkyl, gegebenenfalls durch Methyl, Ethyl, Methoxy, Ethoxy, Chlor oder Brom substituiertes Phenyl oder Thienyl, X Stickstoff oder den Rest $CR^4$, in dem $R^4$ für Wasserstoff oder Cyano steht, und $R^3$ einen Rest der Formel

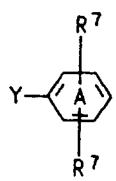,

bedeuten, wobei Y, $R^7$ und der Ring A jeweils die in Anspruch 1 genannte Bedeutung besitzen.

**3.** Thienonverbindungen gemäß Anspruch 1, dadurch gekennzeichnet, daß $R^1$ Cyano, $R^2$ Methyl, gegebenenfalls durch Methoxy substituiertes Phenyl oder Thienyl, X Stickstoff oder den Rest $CR^4$, in dem $R^4$ für Wasserstoff oder, Cyano steht, und $R^3$ einen Rest der Formel

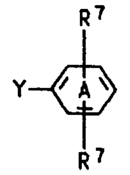,

bedeuten, wobei der Ring A nicht benzoanelliert ist und Y und $R^7$ jeweils die in Anspruch 1 genannte Bedeutung besitzen.

**4.** Verwendung der Thienonverbindungen der Formel Ia

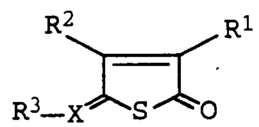 (Ia),

in der

| | |
|---|---|
| $R^1$ | Cyano, $C_1$-$C_4$-Alkoxycarbonyl, Carbamoyl oder $C_1$-$C_4$-Mono-oder Dialkylcarbamoyl, |
| $R^2$ | Wasserstoff, $C_1$-$C_8$-Alkyl, gegebenenfalls durch $C_1$-$C_4$-Alkyl, $C_1$-$C_4$-Alkoxy, Chlor, Brom oder Nitro substituiertes Phenyl, Furyl, Thienyl oder Halogen, |
| X | Stickstoff oder den Rest $CR^4$, in dem $R^4$ für Wasserstoff, $C_1$-$C_4$-Alkyl, $C_1$-$C_4$-Alkoxycarbonyl, Cyano oder gegebenenfalls durch $C_1$-$C_4$-Alkyl, $C_1$-$C_4$-Alkoxy, Chlor, Brom oder Nitro substituiertes Phenyl steht, und |
| $R^3$ | einen Rest der Formel |

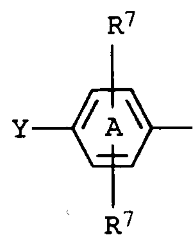, 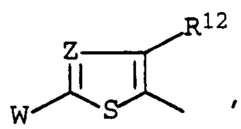, 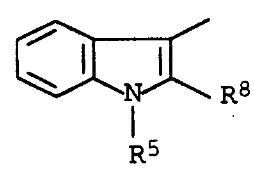,

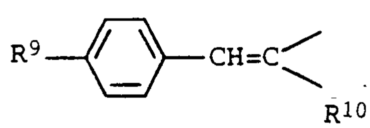, 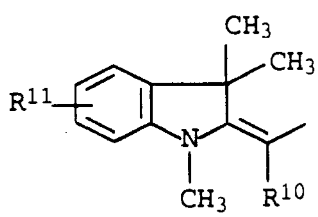 oder 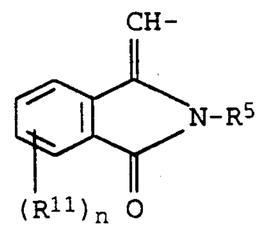

| | |
|---|---|
| | bedeuten, wobei |
| | der Ring A gegebenenfalls benzoanelliert ist, |
| Y | für $NR^5R^6$ oder $OR^8$, |
| $R^5$ und $R^6$ | gleich oder verschieden sind und unabhängig voneinander jeweils für Wasserstoff, $C_1$-$C_8$-Alkyl, das gegebenenfalls durch Hydroxy, Chlor, Cyano, $C_1$-$C_4$-Alkoxy, $C_1$-$C_4$-Alkanoyloxy, $C_1$-$C_4$-Alkoxycarbonyloxy oder Phenyl substituiert ist, Allyl, $C_5$-$C_7$-Cycloalkyl oder Phenyl oder zusammen mit dem sie verbindenden Stickstoffatom für einen 5- oder 6-gliedrigen gesättigten heterocyclischen Ring, |
| $R^8$ | $C_1$-$C_4$-Alkyl oder Phenyl, |
| $R^7$ | unabhängig voneinander jeweils für Wasserstoff, Chlor, Brom, Hydroxy, $C_1$-$C_4$-Alkyl, |

$C_1$-$C_4$-Alkoxy, gegebenenfalls durch Chlor, $C_1$-$C_4$-Alkoxy oder Phenoxy substituiertes $C_1$-$C_4$-Alkanoylamino, Benzoylamino, $C_1$-$C_4$-Mono- oder Dialkylaminosulfonylamino, $C_1$-$C_4$-Alkanoyloxy oder Phenylsulfonyloxy,

$R^9$ für Wasserstoff, $C_1$-$C_4$-Dialkylamino oder $C_1$-$C_4$-Alkoxy,

$R^{10}$ für Wasserstoff oder Cyano,

$R^{11}$ für Wasserstoff, Chlor, Brom oder $C_1$-$C_4$-Alkyl,

$R^{12}$ für Wasserstoff, Chlor, Brom, $C_1$-$C_8$-Alkyl, gegebenenfalls durch $C_1$-$C_4$-Alkyl, $C_1$-$C_4$-Alkoxy, Chlor, Brom oder Nitro substituiertes Phenyl oder Thienyl,

W für den Rest $NR^5R^6$ oder

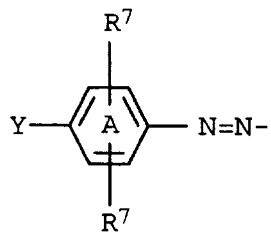

,

wobei $R^5$, $R^6$, $R^7$, Y und der Ring A jeweils die obengenannte Bedeutung besitzen,

Z für Stickstoff oder den Rest $CR^1$, worin $R^1$ die obengenannte Bedeutung besitzt, und

n für 1, 2, 3 oder 4 stehen,

zum Färben von Fasern.

## Claims

**1.** A thienone compound of the formula I where

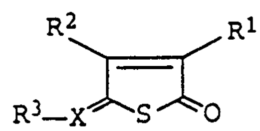

(I),

$R^1$ is cyano, $C_1C_4$-alkoxycarbonyl, carbamoyl or $C_1$-$C_4$-monoalkylcarbamoyl or -dialkylcarbamoyl,

$R^2$ is hydrogen, $C_1$-$C_8$-alkyl, unsubstituted or $C_1$-$C_4$-alkyl-, $C_1$-$C_4$-alkoxy-, chlorine-, bromine- or nitrosubstituted phenyl, furyl, thienyl or halogen,

X is nitrogen or $CR^4$ where $R^4$ is hydrogen, $C_1$-$C_4$-alkyl, $C_1$-$C_4$-alkoxycarbonyl, cyano or unsubstituted or $C_1$-$C_4$-alkyl-, $C_1$-$C_4$-alkoxy-, chlorine-, bromine- or nitrosubstituted phenyl, and is a radical of the formula

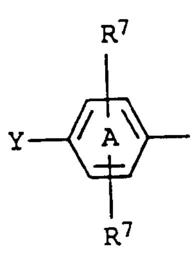

,

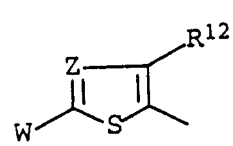

,

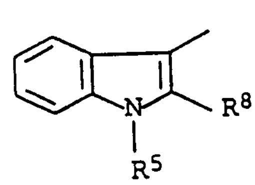

,

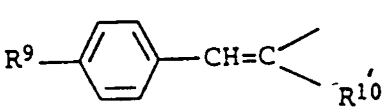

,

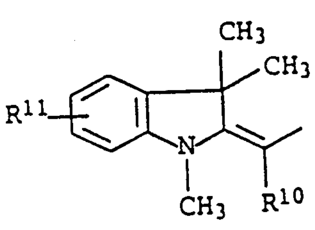

oder

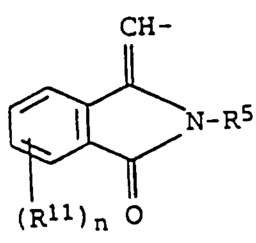

where the ring A may be benzofused,

Y is $NR^5R^6$ or $OR^8$

$R^5$ and $R^6$ are identical or different and each is independently of the other hydrogen, $C_1$-$C_8$-alkyl which is unsubstituted or substituted by hydroxyl, chlorine, cyano, $C_1$-$C_4$-alkoxy, $C_1$-$C_4$-alkanoyloxy, $C_1$-$C_4$-alkoxycarbonyloxy or phenyl, allyl, $C_5$-$C_7$-cycloalkyl or phenyl or together with the nitrogen atom joining them are a 5- or 6-membered saturated heterocyclic ring

$R^8$ is $C_1$-$C_4$-alkyl or phenyl,

the R's are each independently of one another hydrogen, chlorine, bromine, hydroxyl, $C_1$-$C_4$-alkyl, $C_1$-$C_4$-alkoxy, unsubstituted or chlorine-, $C_1$-$C_4$-alkoxy- or phenoxy-substituted $C_1$-$C_4$-alkanoylamino, benzoylamino, $C_1$-$C_4$-mono- or -dialkylaminosulfonylamino, $C_1$-$C_4$-alkanoyloxy or phenylsulfonyloxy,

$R^9$ is hydrogen, $C_1$-$C_4$-dialkylamino or $C_1$-$C_4$-alkoxy,

$R^{10}$ is hydrogen or cyano,

$R^{11}$ is hydrogen, chlorine, bromine or $C_1$-$C_4$-alkyl,

$R^{12}$ is hydrogen, chlorine, bromine, $C_1$-$C_8$-alkyl, unsubstituted or $C_1$-$C_4$-alkyl-, $C_1$-$C_4$-alkoxy-, chlorine-, bromine- or nitro-substituted phenyl or thienyl,

W is $NR^5R^6$ or

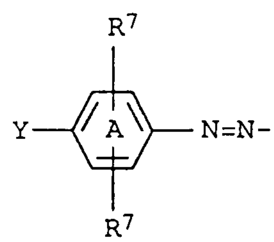

,

where $R^5$, $R^6$, $R^7$, Y and the ring A are each as defined above,

Z is nitrogen or $CR^1$ where $R^1$ is as defined above, and

n is 1, 2, 3 or 4,

with the proviso that if $R^1$ is cyano, X is CH and $R^3$ is 4-dimethylaminophenyl or styryl, then $R^2$ is not methyl or phenyl and that if $R^1$ is cyano, X is CH and $R^3$ is 4-methoxyphenyl, then $R^2$ is not phenyl.

**2.** A thienone compound as claimed in claim 1, wherein $R^1$ is cyano or carbamoyl, $R^2$ is $C_1$-$C_4$-alkyl, unsubstituted or methyl-, ethyl-, methoxy-, ethoxy-, chlorine- or bromine-substituted phenyl or thienyl, X is nitrogen or $CR^4$ where $R^4$ is hydrogen or cyano, and $R^3$ is a radical of the formula

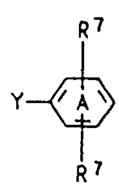

,

where Y, $R^7$ and the ring A are each as defined in claim 1.

**3.** A thienone compound as claimed in claim 1, wherein $R^1$ is cyano, $R^2$ is methyl, unsubstituted or methoxy-substituted phenyl or thienyl, X is nitrogen or $CR^4$ where $R^4$ is hydrogen or cyano, and $R^3$ is a radical of the formula

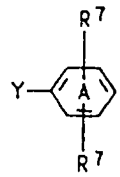

,

where the ring A is not benzofused and Y and $R^7$ are each as defined in claim 1.

**4.** The use of a thienone compound of the formula Ia

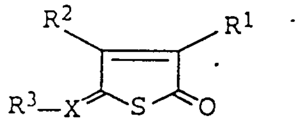

(Ia),

where

| | |
|---|---|
| $R^1$ | is cyano, $C_1$-$C_4$-alkoxycarbonyl, carbamoyl or $C_1$-$C_4$-monoalkylcarbamoyl or -dialkylcarbamoyl, |
| $R^2$ | is hydrogen, $C_1$-$C_8$-alkyl, unsubstituted or $C_1$-$C_4$-alkyl-, $C_1$-$C_4$-alkoxy-, chlorine-, bromine- or nitro-substituted phenyl, furyl, thienyl or halogen, |
| X | is nitrogen or $CR^4$ where $R^4$ is hydrogen, $C_1$-$C_4$-alkyl, $C_1$-$C_4$-alkoxycarbonyl, cyano or unsubstituted or $C_1$-$C_4$-alkyl-, $C_1$-$C_4$-alkoxy-, chlorine-, bromine- or nitrosubstituted phenyl, and |
| $R^3$ | is a radical of the formula |

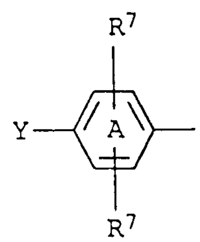

,

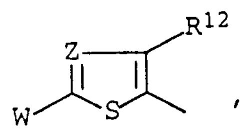

,

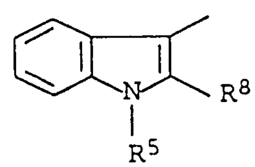

,

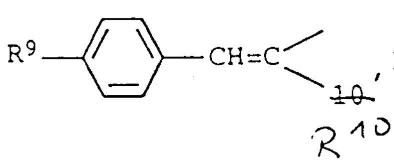

,

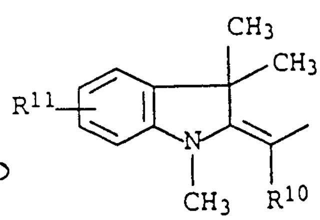

oder

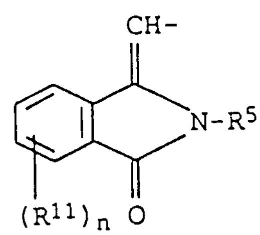

| | |
|---|---|
| | where the ring A may be benzofused, |
| Y | is $NR^5R^6$ or $OR^8$ |
| $R^5$ and $R^6$ | are identical or different and each is independently of the other hydrogen, $C_1$-$C_8$-alkyl which is unsubstituted or substituted by hydroxyl, chlorine, cyano, $C_1$-$C_4$-alkoxy, $C_1$-$C_4$-alkanoyloxy, $C_1$-$C_4$-alkoxycarbonyloxy or phenyl, allyl, $C_5$-$C_7$-cycloalkyl or phenyl or together with the nitrogen atom joining them are a 5- or 6-membered saturated heterocyclic ring |
| $R^8$ | is $C_1$-$C_4$-alkyl or phenyl, |
| the $R^7$s | are each independently of one another hydrogen, chlorine, bromine, hydroxyl, $C_1$-$C_4$-alkyl, $C_1$-$C_4$-alkoxy, unsubstituted or chlorine-, $C_1$-$C_4$-alkoxy- or phenoxy-substituted $C_1$-$C_4$-alkanoyl-amino, benzoylamino, $C_1$-$C_4$-mono- or -dialkylaminosulfonylamino, $C_1$-$C_4$-alkanoyloxy or phenylsulfonyloxy, |
| $R^9$ | is hydrogen, $C_1$-$C_4$-dialkylamino or $C_1$-$C_4$-alkoxy, |

| | |
|---|---|
| $R^{10}$ | is hydrogen or cyano, |
| $R^{11}$ | is hydrogen, chlorine, bromine or $C_1$-$C_4$-alkyl, |
| $R^{12}$ | is hydrogen, chlorine, bromine, $C_1$-$C_8$-alkyl, unsubstituted or $C_1$-$C_4$-alkyl-, $C_1$-$C_4$-alkoxy-, chlorine-, bromine- or nitro-substituted phenyl or thienyl, |
| W | is $NR^5R^6$ or |

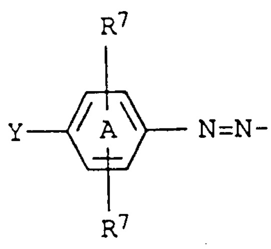

,

where $R^5$, $R^6$, $R^7$, Y and the ring A are each as defined above,

| | |
|---|---|
| Z | is nitrogen or $CR^1$ where $R^1$ is as defined above, and |
| n | is 1, 2, 3 or 4, |

for dyeing fibers.

## Revendications

**1.** Dérivés de thiénone de formule I

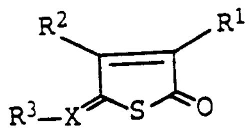

(I),

dans laquelle

| | |
|---|---|
| $R^1$ | représente un reste cyano, (alcoxy en $C_1$-$C_4$)carbonyle, carbamoyle ou mono- ou di-(alkyl en $C_1$-$C_4$)carbamoyle, |
| $R^2$ | représente un atome d'hydrogène ou un reste alkyle en $C_1$-$C_8$, phényle éventuellement substitué par un groupement alkyle en $C_1$-$C_4$, alcoxy en $C_1$-$C_4$, chloro, bromo ou nitro, un reste furyle, thiényle ou un atome d'halogène, |
| X | représente un atome d'azote ou le reste $CR^4$, dans lequel $R^4$ est mis pour un atome d'hydrogène ou pour un radical alkyle en $C_1$-$C_4$, (alcoxy en $C_1$-$C_4$)carbonyle, cyano ou phényle éventuellement substitué par un groupement alkyle en $C_1$-$C_4$, alcoxy en $C_1$-$C_4$, chloro, bromo au nitro, et |
| $R^3$ | représente un reste de formule |

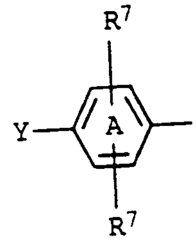 , 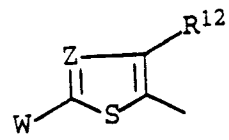 , 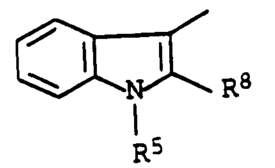 ,

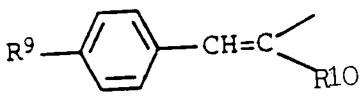 , 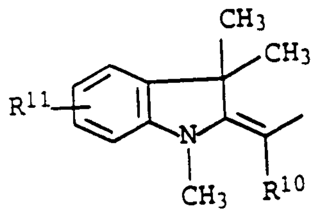 ou 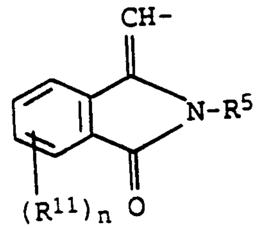

où

le noyau A est éventuellement benzocondensé,

Y est mis pour $NR^5R^6$ ou $OR^8$,

$R^5$ et $R^6$ étant identiques ou différents et représentant chacun, indépendamment l'un de l'autre, un atome d'hydrogène, un reste alkyle en $C_1$-$C_8$ qui est éventuellement substitué par un groupement hydroxy, chloro, cyano, alcoxy en $C_1$-$c_4$, alcanoyloxy en $c_1$-$c_4$, (alcoxy en $C_1$-$C_4$)carbonyloxy ou phényle, un reste allyle, cycloalkyle en $C_5$-$C_7$ ou phényle, ou formant ensemble, avec l'atome d'azote qui les relie, un noyau hétérocyclique saturé à 5 ou 6 maillons,

$R^8$ étant mis pour un reste alkyle en $C_1$-$C_4$ ou phényle,

$R^7$ est mis chaque fois et indépendamment pour un atome d'hydrogène, de chlore, de brome, pour un reste hydroxy, alkyle en $C_1$-$C_4$, alcoxy en $C_1$-$C_4$, (alcanoyl en $C_1$-$C_4$)-amino éventuellement substitué par un groupement chloro, alcoxy en $C_1$-$C_4$ ou phénoxy, pour un reste benzoylamino, mono- ou di(alkyl en $C_1$-$C_4$)aminosulfonylamino, alcanoyloxy en $C_1$-$C_4$ ou phénylsulfonyloxy,

$R^9$ est mis pour un atome d'hydrogène ou pour un reste di(alkyl en $C_1$-$C_4$)amino ou alcoxy en $C_1$-$C_4$,

$R^{10}$ est mis pour un atome d'hydrogène ou un reste cyano,

$R^{11}$ est mis pour un atome d'hydrogène, de chlore, de brome ou pour un reste alkyle en $C_1$-$C_4$,

$R^{12}$ est mis pour un atome d'hydrogène, de chlore, de brome ou pour un reste alkyle en $C_1$-$C_8$, phényle éventuellement substitué par un groupement alkyle en $C_1$-$C_4$, alcoxy en $C_1$-$C_4$, chloro, bromo ou nitro ou pour un reste thiényle,

W est mis pour le reste $NR^5R^6$ ou

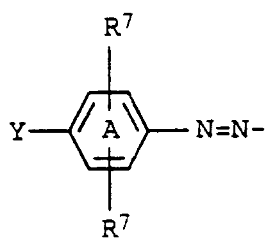 ,

$R^5$, $R^6$, $R^7$, Y et le noyau A ayant chacun la signification sus-indiquée,

Z est mis pour un atome d'azote ou pour le reste $CR^1$, $R^1$ ayant la signification sus-indiquée, et

n est mis pour 1, 2, 3 ou 4,

étant spécifié que quand $R^1$ est mis pour un reste cyano, que X est mis pour le reste CH et que $R^3$ est mis pour le reste 4-diméthylaminophényle ou styryle, $R^2$ ne représente pas un radical méthyle ou phényle, et que quand $R^1$ est mis pour un reste cyano, que X est mis pour le reste CH et que $R^3$ est

mis pour le reste 4-méthoxyphényle, $R^1$ ne représente pas un radical phényle.

**2.** Dérivés de thiénone selon la revendication 1, caractérisés en ce que $R^1$ représente un reste cyano ou carbamoyle, $R^2$ représente un reste alkyle en $C_1$-$C_4$, un reste phényle éventuellement substitué par un groupement méthyle, éthyle, méthoxy, éthoxy, chloro ou bromo ou un reste thiényle, X représente un atome d'azote ou le reste $CR^4$, dans lequel $R^4$ est mis pour un atome d'hydrogène au un groupement cyano, et $R^3$ est un reste de formule

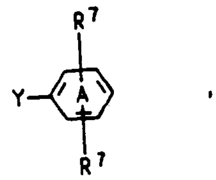

Y, $R^7$ et le noyau A ayant chacun la signification indiquée dans la revendication 1.

**3.** Dérivés de thiénone selon la revendication 1, caractérisés en ce que $R^1$ représente un reste cyano, $R^2$ représente un reste méthyle, un reste phényle éventuellement substitué par un groupement méthoxy ou un reste thiényle, X représente un atome d'azote ou le reste $CR^4$, dans lequel $R^4$ est mis pour un atome d'hydrogène ou un groupement cyano, et $R^3$ est un reste de formule

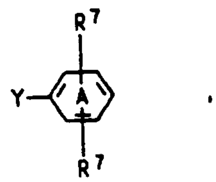

le noyau A n'étant pas benzocondensé et Y et $R^7$ ayant chacun la signification indiquée dans la revendication 1.

**4.** Utilisation, pour la teinture de fibres, des dérivés de thiénone de formule Ia

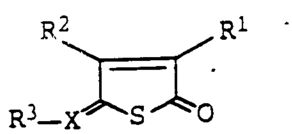

(Ia),

dans laquelle

- $R^1$ représente un reste cyano, (alcoxy) en $C_1$-$C_4$)carbonyle, carbamoyle ou mono- ou di-(alkyl en $C_1$-$C_4$)carbamoyle,
- $R^2$ représente un atome d'hydrogène ou un reste alkyle en $C_1$-$C_8$, phényle éventuellement substitué par un groupement alkyle en $C_1$-$C_4$, alcoxy en $C_1$-$C_4$, chloro, bromo ou nitro, pour un reste furyle, thiényle ou pour un atome d'halogène,
- X représente un atome d'azote ou le reste $CR^4$, dans lequel $R^4$ est mis pour un atome d'hydrogène ou pour un radical alkyle en $C_1$-$C_4$, (alcoxy en $C_1$-$C_4$)carbonyle, cyano ou phényle éventuellement substitué par un groupement alkyle en $C_1$-$C_4$, alcoxy en $C_1$-$C_4$, chloro, bromo ou nitro, et
- $R^3$ représente un reste de formule

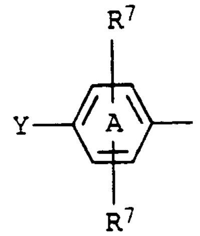 , 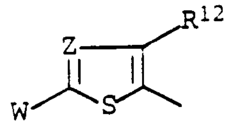 , 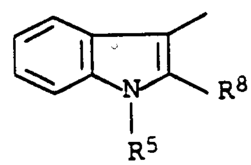 ,

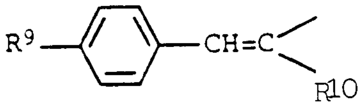 , 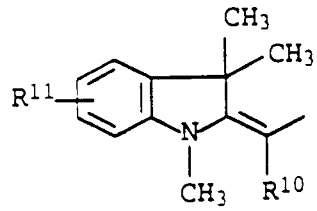 ou 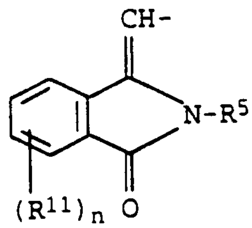

où

le noyau A est éventuellement benzocondensé,

| | |
|---|---|
| Y | est mis pour $NR^5R^6$ ou $OR^8$, |
| $R^5$ et $R^6$ | étant identiques ou différents et représentant chacun, indépendamment l'un de l'autre, un atome d'hydrogène, un reste alkyle en $C_1$-$C_8$ qui est éventuellement substitué par un groupement hydroxy, chloro, cyano, alcoxy en $C_1$-$C_4$, alcanoyloxy en $C_1$-$C_4$, (alcoxy en $C_1$-$C_4$)carbonyloxy ou phényle, un reste allyle, cycloalkyle en $C_5$-$C_7$ ou phényle, ou formant ensemble, avec l'atome d'azote qui les relie, un noyau hétérocyclique saturé à 5 ou 6 maillons, |
| $R^9$ | étant mis pour un reste alkyle en $C_1$-$C_4$ ou phényle, |
| $R^7$ | est mis chaque fois et indépendamment pour un atome d'hydrogène, de chlore, de brome, pour un reste hydroxy, alkyle en $C_1$-$C_4$, alcoxy en $C_1$-$C_4$, (alcanoyl en $C_1$-$C_4$)-amino éventuellement substitué par un groupement chloro, alcoxy en $C_1$-$C_4$ ou phénoxy, pour un reste benzoylamino, mono- ou di(alkyl en $C_1$-$C_4$)aminosulfonylamino, alcanoyloxy en $C_1$-$C_4$ ou phénylsulfonyloxy, |
| $R^9$ | est mis pour un atome d'hydrogène ou pour un reste di(alkyl en $C_1$-$C_4$)amino ou alcoxy en $C_1$-$C_4$, |
| $R^{10}$ | est mis pour un atome d'hydrogène ou un reste cyano, |
| $R^{11}$ | est mis pour un atome d'hydrogène, de chlore, de brome ou pour un reste alkyle en $C_1$-$C_4$, |
| $R^{12}$ | est mis pour un atome d'hydrogène, de chlore, de brome ou pour un reste alkyle en $C_1$-$C_8$, phényle éventuellement substitué par un groupement alkyle en $C_1$-$C_4$, alcoxy en $C_1$-$C_4$, chloro, bromo ou nitro ou pour un reste thiényle, |
| W | est mis pour le reste $NR^5R^6$ ou |

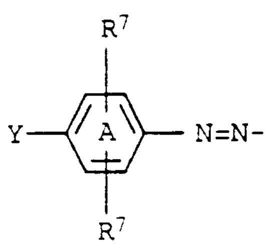 ,

$R^5$, $R^6$, $R^7$, Y et le noyau A ayant chacun la signification sus-indiquée,

| | |
|---|---|
| Z | est mis pour un atome d'azote ou pour le reste $CR^1$, $R^1$ ayant la signification sus-indiquée, et |
| n | est mis pour 1, 2, 3 ou 4. |